# FASCICULE DE BREVET EUROPEEN

(11) **EP 2 350 605 B1**
(45) Date de publication et mention de la délivrance du brevet: **25.04.2018**
(21) Numéro de dépôt: 09768179.5
(22) Date de dépôt: 11.11.2009
(51) Int. Cl.: G01N 1/08, G01N 1/20, G01N 33/00

(54) **DISPOSITIF ET PROCÉDÉ POUR L'ÉCHANTILLONNAGE ET LA RÉCUPÉRATION DE PARTICULES EMPILÉES**
VORRICHTUNG UND VERFAHREN ZUR PROBENAHME UND GEWINNUNG GESTAPELTER PARTIKEL
DEVICE AND METHOD FOR SAMPLING AND RECOVERING STACKED PARTICLES

(30) Priorité: 12.11.2008 FR 0806291
(43) Date de publication de la demande: 03.08.2011
(73) Titulaire: Total Raffinage France, 92400 Courbevoie (FR)
(72) Inventeur: LEROY, Pascal, F-76290 Montivilliers (FR); COTTARD, Bernard, F-76430 Saint Romain de Colbosc (FR); PINON, Ulysse, F-76600 Le Havre (FR)
(74) Mandataire: Largeau, Béatrice
(86) Numéro de dépôt international: PCT/FR2009/052172
(87) Numéro de publication internationale: WO 2010/055262

(56) Documents cités:
- EP-A- 0 251 411
- FR-A- 2 161 170
- FR-A- 2 566 530
- FR-A- 2 679 655
- FR-A- 2 872 284
- GB-A- 1 538 652
- US-A- 3 233 463
- US-A- 4 072 059
- US-A1- 2005 262 928

## Description

L'invention concerne un dispositif et un procédé pour l'échantillonnage et/ou la récupération de matières solides disposées à l'état particulaire en une ou plusieurs couches.

L'invention s'applique plus particulièrement à tout type de matières solides, en poudre ou en grains, comme par exemple des céréales stockées en silos dont on veut connaître l'état de fermentation, les sables, les gravillons, les charbons, ciments ou autres matières solides, dont on veut contrôler la qualité des grains et/ou leurs propriétés physico-chimiques représentatives de leur disposition et/ou de leur empilement, par exemple dans une enceinte.

Plus précisément, l'invention s'applique à l'échantillonnage et/ou la récupération de grains de catalyseurs dans une enceinte de type réacteur chimique, par exemple en vue de réaliser, différentes analyses et/ou tests physico-chimiques. Ces analyses et/ou test peuvent permettre de déterminer si le catalyseur doit être changé en tout ou partie, ou mesurer son état d'activité, ou encore évaluer la quantité dudit catalyseur à envoyer en régénération.

En effet, ces analyses et/ou tests physico-chimiques effectués sur des grains de catalyseur prélevés dans le lit fixe d'un réacteur chimique, par exemple l'évaluation des teneurs en métaux, en carbone ou différents polluants, permettent de vérifier si le catalyseur a subi des modifications lors de son transport, de sa manutention au cours de chargements dans des futs, des conteneurs ou in fine, dans ledit réacteur. Ils permettent aussi de contrôler si les grains de catalyseur ont subi un vieillissement pendant le cycle de leur utilisation en présence d'un hydrocarbure, un tassement, voire une pollution, un empoisonnement, ou encore un colmatage.

Plus particulièrement, c'est à cette dernière application qui consiste à échantillonner et/ou prélever des grains de catalyseur dans un lit fixe de réacteur chimique, que l'on se référera dans la suite de la présente description, cependant le dispositif et son procédé associé, objets de la présente invention, s'appliquent à tout type d'échantillonnage et/ou de récupération de matières solides disposées à l'état particulaire.

Il n'est pas aisé de prélever un échantillon représentatif dans un empilement de particules sans affecter gravement l'ordre dudit empilement. Une solution connue est de faire un ou plusieurs prélèvement(s) à l'aide d'un tube que l'on fait pénétrer dans l'épaisseur des particules solides, notamment sur toute sa hauteur.

Des dispositifs permettant l'échantillonnage et/ou la récupération de particules solides empilées par cette dernière technique, encore appelée « carottage » sont connus et décrits dans plusieurs dizaines de brevets.

D'autres dispositifs n'utilisant pas le carottage mais fonctionnant par aspiration d'air sont divulgués, par exemple dans US 4 072 059 ou GB 1 538 652. Ces dispositifs peuvent présenter l'inconvénient de déplacer et de prélever la matière solide, non seulement à leur extrémité, mais également autour de l'ensemble de la zone de prélèvement, notamment quand la sonde de prélèvement comporte des lumières en périphérie pour améliorer l'aspiration. De plus, la limite du dispositif est rapidement atteinte quand la couche supérieure du catalyseur est dure, par exemple cokée, ledit dispositif ne pouvant pas facilement pénétrer dans le lit de catalyseur.

Un autre dispositif pour l'échantillonnage et/ou la récupération de grains de catalyseur, fonctionnant par fluidisation, est décrit dans le fascicule du brevet Français FR 2 566 530 de la présente Demanderesse. Le dispositif comprend deux tubes coaxiaux raccordés l'un à l'autre de façon étanche dans lequel l'espace annulaire séparant les tubes est connecté à un fluide sous pression afin que des particules solides soient collectées par l'extrémité inférieure du tube interne et acheminées par l'intérieur dudit tube jusqu'à un collecteur. Le document US2005/0262928 décrit un embout annulaire muni de rainures hélicoïdales pour guider un gaz sous pression à l' extrémité d' un appareil de collecte d' échantillons. Tous ces dispositifs, disponibles dans l'Art, présentent un ou plusieurs inconvénient(s), ils peuvent être insuffisants par exemple en termes de :
- vitesse ou débit de prélèvement,
- précision dudit prélèvement,
- capacité de pénétration du lit fixe de catalyseur, notamment quand celui-ci est gras, et/ou présentant des grains de catalyseur tassés, amalgamés et/ou cokés,
- capacité pour remonter les particules collectées, en particulier lorsque le collecteur est situé au-dessus de la sonde de prélèvement,
- préservation des particules (prélèvement sans casse excessive de particules), et/ou
- représentativité du prélèvement.

Le but de la présente invention vise à remédier en tout ou partie à ces inconvénients en proposant un dispositif et un procédé associé permettant d'échantillonner et/ou récupérer tout type de particules solides disposées à l'état particulaire en une ou plusieurs couches.

Selon un premier aspect, l'invention a pour objet un dispositif pour l'échantillonnage et/ou la récupération de matières solides empilées à l'état particulaire, comprenant une sonde de prélèvement connectée à un collecteur d'échantillon, le dispositif étant caractérisé en ce que la sonde comprend ou est constituée de :
- au moins deux tubes coaxiaux, un tube interne et un tube externe, raccordés étanches à leur partie supérieure et présentant des parties inférieures aptes à pénétrer dans l'empilement des matières solides,
- un espace annulaire entre les deux tubes auquel est connectée une source de fluide sous pression,
- une connexion de l'extrémité supérieure du tube interne au collecteur d'échantillons pour y acheminer les particules prélevées grâce notamment à l'action du fluide sous pression,
- une rampe hélicoïdale comprenant au moins une portion de spire fixée et enroulée autour de la partie inférieure externe du tube interne et dont la largeur de la spire est sensiblement égale à la différence des rayons interne du tube externe et externe du tube interne.

Le dispositif selon l'invention permet de réaliser plus facilement des prélèvements de matières solides, en particulier dans le cas de catalyseur, notamment des grains de catalyseur chargés dense et disposés en lit fixe, par exemple, en fin de cycle de fonctionnement, c'est-à-dire lorsque les catalyseurs sont cokés, tassés et/ou gras. Sans vouloir être lié par cette théorie, le fluide vecteur sous pression canalisé par la rampe hélicoïdale engendre près de l'extrémité inférieure de la sonde de prélèvement un mouvement tourbillonnaire facilitant le décollement et la remontée des particules. Tout particulièrement le dispositif selon l'invention permet de collecter des échantillons de particules représentatifs de l'empilement, notamment de la hauteur de cet empilement, et avec un minimum de dégradation de ces particules, notamment quant à leur granulométrie.

C'est ainsi que des hauteurs de prélèvement de plusieurs mètres, voire supérieures à dix mètres dans un lit fixe de catalyseur sont accessibles avec le dispositif, objet de l'invention ; la longueur de la sonde étant dans ce dernier cas adaptée à la hauteur du lit par ajout de plusieurs allonges, reliées étanches successivement entres elles et fixées sur ladite sonde.

Par rapport à l'Art, les prélèvements et/ou échantillonnages de matières solides avec le dispositif de la présente invention sont plus rapides, même en milieu difficile, et surtout plus facilement réalisables grâce à une meilleure efficacité du dispositif.

Le dispositif objet de la présente invention peut permettre également le remplissage de particules en lieu et place de la matière solide prélevée. Ce remplissage par du catalyseur neuf, régénéré ou autre, intervient par le tube central quand le prélèvement de grains de catalyseur est terminé.

Les particules échantillonnées et/ou prélevées par le dispositif peuvent être de tout type, tels des grains de catalyseur, des billes, etc. Les particules peuvent également être de toute taille. Cependant, lesdites particules présentent en général une longueur comprise entre 0,5 mm et 50 mm, et de préférence entre 1 mm et 15 mm, ainsi qu'un diamètre compris entre 0,2 mm et 10 mm, et de préférence, compris entre 0,5 mm et 6 mm.

Ces particules peuvent être, par exemple et non limitativement, des grains de catalyseurs, des charbons, des céréales, des ciments, des sables, des graviers, des minerais et/ou des composés chimiques ou plastiques.

Elles peuvent être empilées dans une enceinte, tel un réacteur, un silo, un wagon, un camion, ou bien se présenter sous forme de tas, de piles, de terril ou de structures équivalentes.

Les particules à prélever peuvent être présentes dans un milieu gazeux ou liquide.

Le collecteur de particules, connecté à la partie supérieure du tube interne, est alimenté, via le tube central, par l'arrivée des grains de catalyseur fluidisé par l'intermédiaire du fluide sous pression.

Ce collecteur peut comprendre en son intérieur une matière élastique de telle façon que les particules collectées ne cassent pas ou peu lors de leur impact dans ledit collecteur et ne soient pas entraînées avec les poussières de catalyseur.

Une sortie pour ces poussières mélangées au fluide sous pression peut être aménagée dans le collecteur. Disposé en aval dudit collecteur, un cyclone peut permettre ensuite de séparer et orienter différemment les poussières et ledit fluide sous pression.

Le collecteur peut présenter une taille suffisante pour rassembler l'ensemble de l'échantillon souhaité.

Selon une variante, le collecteur ne présente pas une telle taille, mais des parties de l'échantillon sont collectées successivement dans des récipients identifiés et adaptés à une sortie dédiée du collecteur au fur et à mesure du prélèvement.

L'extrémité inférieure du tube interne, sur laquelle est installée la rampe hélicoïdale, peut être en retrait de l'extrémité inférieure du tube externe. Cette disposition peut permettre, en limitant au fluide sous pression de diffuser à l'extérieur du tube externe, d'améliorer la capacité d'aspiration des particules et permettre audit fluide sous pression de fluidiser les particules immédiatement placées sous la sonde, et d'entraîner ces particules dans le tube interne vers le collecteur de matières solides.

Tout particulièrement ce retrait peut varier de 1 à 50 mm, et de préférence de 1 à 30 mm.

Cette extrémité inférieure du tube externe peut présenter une faible épaisseur, et notamment être biseautée vers l'intérieur, et/ou vers l'extérieur sur une hauteur comprise entre 1 mm et 50 mm, et de préférence entre 5 mm et 30 mm, par exemple de façon que l'extrémité du tube soit effilée afin de permettre une meilleure pénétration de la sonde dans l'empilement de particules. Un autre effet obtenu avec l'extrémité du tube externe en forme de biseau est de limiter la sortie du fluide sous pression à l'extérieur dudit tube externe, c'est-à-dire dans l'empilement de particules, hors zone de prélèvement des dites particules. De plus, cette même extrémité biseautée peut être configurée de telle façon que le fluide sous pression soit dirigé à la sortie de l'espace annulaire vers l'axe central des tubes par un aménagement particulier de l'extrémité inférieure du tube externe.

Tout particulièrement, des cales peuvent être positionnées entre l'intérieur du tube externe et l'extérieur du tube interne, notamment afin que la distance annulaire soit sensiblement la même sur une circonférence donnée et sur toute la longueur de la sonde.

Le rapport entre le rayon interne du tube externe et le rayon externe du tube interne peut varier de 1,1 à 30 et de préférence 1,2 à 5.

Dans chacune des configurations d'associations des tubes internes et externes, le tube externe est connecté à une arrivée de fluide sous pression, encore appelé gaz vecteur, qui peut être de l'air ou un gaz neutre, tel que l'azote.

La pression de ce fluide peut varier de 0 à 25 bars, de préférence entre 0,5 à 25 bars et de préférence entre 1 et 10 bars.

Bien entendu, plus la hauteur entre l'extrémité inférieure de la sonde et l'extrémité supérieure de ladite sonde ou du collecteur est grande, plus la pression du fluide doit être importante. Par exemple lorsque cette hauteur est d'une dizaine de mètres, la pression peut varier de 10 à 15 bars.

La rampe hélicoïdale disposée en partie inférieure du tube interne peut diviser l'espace annulaire en au moins deux canaux, en particulier quatre canaux ou plus, en fonction du nombre de spires, ou de portions de spires installées. L'impact du fluide sur le décollement des grains de catalyseur puis la mise en mouvement de ces grains à l'extrémité inférieure du tube externe, sera d'autant plus grand que le nombre de spires est important, c'est-à-dire un nombre de canaux d'autant plus important.

La hauteur de la rampe hélicoïdale peut être comprise entre 50 mm et la hauteur du tube interne recouvert par le tube externe et de préférence entre 150 mm et 500 mm.

L'espace annulaire, c'est-à-dire la largeur de la spire de la rampe hélicoïdale, est sensiblement égal à la distance comprise entre les tubes interne et externe.

L'épaisseur de la spire est comprise entre 0,1 mm et 50 mm, et de préférence, entre 1 et 10 mm.

Par « largeur de la spire », on entend au sens de la présente invention la distance séparant la partie la plus lointaine de la spire de la paroi externe de la rampe hélicoïdale.

Par « épaisseur de la spire », on entend au sens de la présente invention la distance séparant les parois de la spire perpendiculaires à la rampe hélicoïdale.

Le pas de la spire est compris entre 10 mm et 300 mm, et de préférence, entre 30 mm et 100 mm.

Le pas peut être également progressif, c'est-à-dire présentant des pas variables sur un même équipement.

Cette partie hélicoïdale peut être installée sur la partie inférieure de la sonde de manière irréversible.

Selon une autre variante, cette partie est montée amovible par rapport à la partie inférieure de la dernière allonge de la sonde. Elle peut notamment être fixée par un pas de vis complémentaire présent sur la partie externe du tube intérieur. Dans ce cas la fixation doit être étanche sur le tube interne.

Le cas du montage amovible est particulièrement avantageux puisque cela permet d'adapter la rampe par rapport à l'état des particules, au type des particules et/ou à l'état de leur empilement.

Ainsi, une seule sonde avec plusieurs parties amovibles disponibles permet des prélèvements dans de nombreux types de conditions.

Avantageusement, la partie interne du tube externe recouvrant la rampe hélicoïdale contient une rainure disposée sensiblement au milieu de 2 spires et dont le pas est sensiblement égal à celui des spires.

De préférence, le volume de la rainure entre deux spires est égal à celui d'une spire.

Avantageusement et selon un mode de réalisation particulier, le tube interne comprend au moins une lumière disposée dans son épaisseur et dont l'axe longitudinal fait un angle de sensiblement 45° avec l'horizontale. Cette lumière apporte une énergie supplémentaire, par l'intermédiaire de la pression du fluide sous pression, afin de faciliter la montée des particules solides fluidisées dans le tube interne.

Avantageusement et selon un autre mode de réalisation, le dispositif comprend au moins un système complémentaire destiné à augmenter la vitesse des particules dans le tube central, afin d'améliorer leur acheminement au collecteur. Ce système peut être disposé autour du tube interne, juste au-dessus de l'extrémité supérieure du tube externe et en particulier situé à proximité, par exemple à moins de 50 cm, et de préférence à moins de 100 cm du collecteur.

Ce système peut être constitué d'une enceinte étanche, entourant le tube interne, ladite enceinte étant alimentée en un deuxième fluide gazeux, tel de l'air ou un gaz neutre. Le tube interne, recouvert par l'enceinte étanche, comporte au moins une lumière pour la circulation dudit fluide gazeux.

Compte-tenu des dimensions des particules prélevées, de la hauteur du prélèvement, la pression de ce deuxième fluide gazeux peut être différente du fluide utilisé pour la fluidisation des particules et notamment plus élevée.

Selon un second aspect, l'invention a aussi pour objet un procédé de prélèvement et/ou d'échantillonnage de matières solides empilées à l'état particulaire, à l'aide du dispositif selon l'invention caractérisé en ce que
- l'extrémité inférieure de la sonde du dispositif est positionnée verticalement ou obliquement sur les matières solides,
- le fluide gazeux est introduit à la pression désirée entre les deux tubes interne et externe,
- les matières solides sont recueillies dans le récipient collecteur dudit dispositif au fur et à mesure que ladite sonde pénètre dans l'empilement,
- la fin du prélèvement, c'est à dire l'arrêt de la pénétration de la sonde est obtenue par coupure de l'alimentation en fluide gazeux.

Plus particulièrement ce procédé est effectué en continu.

Les matières solides sont recueillies au fur et à mesure de la pénétration de la sonde dans l'empilement des particules.

La sonde peut être introduite dans l'empilement de manière verticale, mais aussi de manière oblique.

Il est également possible de se servir de la sonde pour venir extraire des parties non souhaitées présentes dans un empilement.

Selon un troisième aspect, l'invention a pour objet l'utilisation d'un dispositif selon l'invention pour le prélèvement d'échantillon dans un empilement de particules placé dans une atmosphère gazeuse ou liquide.

### Brève description des figures :

L'invention est maintenant décrite en référence aux dessins annexés, non limitatifs, dans lesquels :
- la figure 1 est une représentation schématique du dispositif selon l'invention destiné à l'échantillonnage et/ou la récupération de matières solides disposées à l'état particulaire en une ou plusieurs couches.
   Lesdites matières solides peuvent être, par exemple et non limitativement, des grains de catalyseur disposés denses dans une enceinte de type réacteur chimique.
- la figure 2 est une représentation schématique de plusieurs types de rampe hélicoïdale pouvant équiper la partie inférieure du tube interne du dispositif selon l'invention.

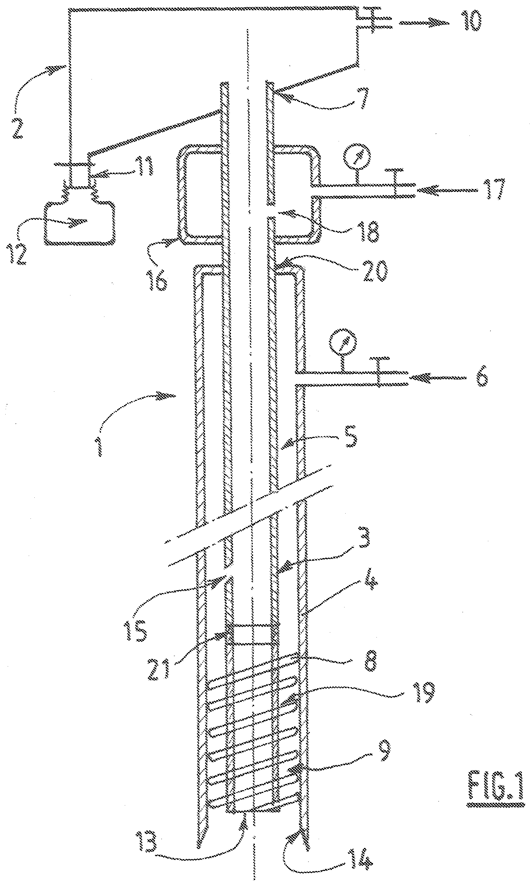

Sur la figure 1 le dispositif est constitué de:
- une sonde de prélèvement (1) dont la partie inférieure est introduite dans l'empilement de matières solides, conformément au procédé objet de la présente invention.
- un collecteur d'échantillons (2) relié mécaniquement à la sonde par l'extrémité supérieure du tube interne (7).

La sonde (1) est constituée de deux tubes coaxiaux, un tube (3), appelé tube interne et disposé à l'intérieur d'un tube (4) dénommé tube externe.

Les tubes interne et externe sont raccordés étanches à leur partie supérieure (20) tandis que leur extrémité inférieure (13 et 14) sont aptes à pénétrer dans l'empilement des matières solides.

L'extrémité inférieure (14) du tube externe est biseauté vers l'intérieur pour une meilleure pénétration de la sonde dans les matières solides.

Entre les deux tubes, un espace annulaire (5) auquel est connectée une source de fluide sous pression (6), généralement du gaz, plus précisément de l'air ou un gaz rare, tel que l'azote eu égard aux propriétés chimiques des matières solides à échantillonner ou récupérer. Des cales (non représentées) d'épaisseur peuvent être positionnées et fixées dans l'espace annulaire pour assurer une distance annulaire entre les deux tubes constante sur toute la longueur de la sonde.

A l'extrémité inférieure du tube interne est vissée, via un pas de vis (21), ou fixée mécaniquement, une rampe hélicoïdale (19) constituée de une ou plusieurs spires (8).

La largeur de la spire (9) est sensiblement égale à la différence de longueur entre les rayons interne du tube extérieur et externe du tube intérieur.

Au moins une lumière (15) est disposée dans l'épaisseur du tube interne afin de faciliter la remontée des matières solides fluidisée à l'intérieur du tube interne (3).

La sonde de prélèvement du dispositif représenté sur la figure 1, comprend un système complémentaire destiné à créer une dépression en partie supérieure du tube interne (3) de la sonde, afin d'améliorer encore la remontée des particules solides dans le collecteur d'échantillons (2).

Ce système est composé d'une enceinte étanche (16) entourant une portion supérieure du tube interne (3), ladite enceinte étant alimentée en un fluide gazeux tel que l'air ou un gaz rare (17). Au moins une lumière (18) est aménagée dans l'épaisseur du tube interne, à l'intérieur dudit système complémentaire pour améliorer par dépression, la remontée des matières solides dans le collecteur d'échantillons.

La sonde de prélèvement (2) comprend une sortie (10) du fluide gazeux entraînant également les éventuelles poussières de matières solides remontées en même temps que lesdites matières solides, ainsi qu'une sortie (11) dédiée aux échantillons de matières solides qui peuvent être récupérés dans un flacon d'échantillonnage (12). En aval de la sortie (10) un cyclone (non représenté) peut être installé pour séparer le gaz des éventuelles poussières.

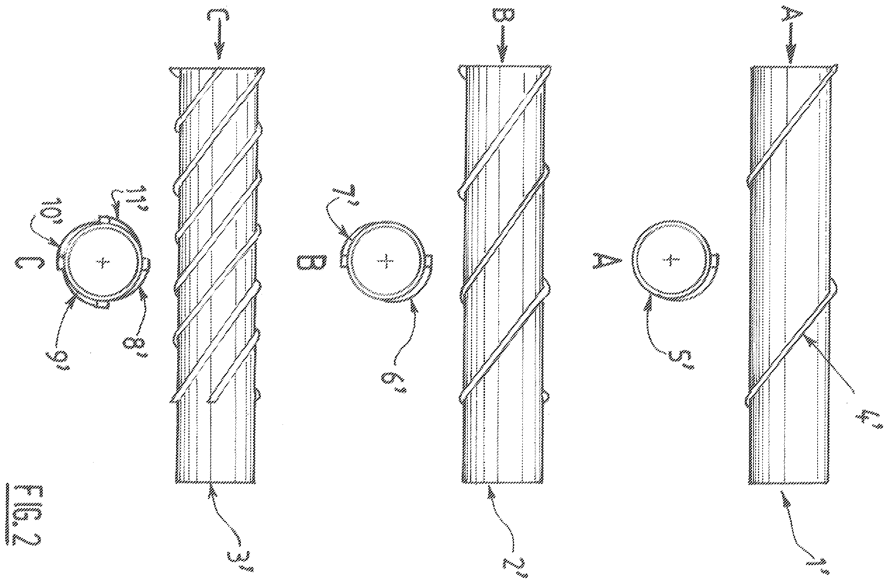

Sur la figure 2 sont représentés trois types de rampe hélicoïdale constituée de 1 (1'), 2 (2') et 4 spires (3').

L'impact du fluide sous pression arrivant à l'extrémité du tube externe dans les matières solides sera d'autant plus important si la rampe hélicoïdale compte 1, 2 ou 4 spires.

En effet, avec 1 spire (4') un seul canal (5') amènera le fluide dans l'empilement des matières solides.

Il y aura, pour une pression donnée, 2 canaux (6' et 7') avec 2 spires et 4 canaux (8', 9', 10' et 11') d'arrivée du fluide sous pression avec 4 spires, permettant de canaliser ledit fluide et lui donner un mouvement tourbillonnaire de type vortex afin d'obtenir un meilleur décollement des matières solides ainsi qu'une meilleure fluidisation pour in fine, améliorer le mouvement de remontée des matières solides dans le tube interne (3'), et donc dans le collecteur d'échantillons.

Un exemple de rampe hélicoïdale réalisée conformément à l'invention et comprenant 4 spires régulièrement et également réparties présente les dimensions suivantes :
- longueur hors tout : 170 mm,
- diamètre externe tube support des spires: 30 mm,
- longueur où sont disposées les spires : 130 mm,
- largeur de chaque spire : 3 mm,
- épaisseur de chacune des spires : 3 mm,
- pas de l'enroulement : 80 mm.

### Exemple

Un dispositif, constitué de sa sonde de prélèvement et de son collecteur d'échantillon est utilisé dans un réacteur chimique pour prélever des échantillons de grains de catalyseur disposés dans un lit fixe à l'intérieur dudit réacteur.

Le catalyseur est constitué majoritairement d'alumine à laquelle ont été ajoutés plusieurs % d'oxydes de Nickel et de molybdène.

Les grains de catalyseur ont la granulométrie moyenne suivante :
- longueur : 4,1 mm
- diamètre : 1,5 mm

La sonde à une longueur hors tout de 1,50 m et est constituée conformément à la présente invention, avec les caractéristiques suivantes :
- rayon externe du tube interne : 17 mm,
- rayon interne du tube interne : 15 mm,
- rayon externe du tube externe : 27 mm,
- rayon interne du tube externe : 25 mm,
- distance de retrait entre les extrémités inférieures des tubes externe et interne : 15 mm,
- l'extrémité inférieure du tube externe est biseautée vers l'intérieur du tube sur une longueur de 10 mm,
- une lumière d'un diamètre de 2 mm est disposée dans l'épaisseur du tube interne à 1 m de l'extrémité inférieure dudit tube et dont l'axe longitudinal fait un angle de 45° avec l'horizontale,
- pas de système complémentaire pour l'augmentation de la vitesse de remontée des particules en direction du collecteur d'échantillons,
- la rampe hélicoïdale comporte 4 spires et ses dimensions sont conformes à celles indiquées ci-dessus.

Deux essais (A et B) sont réalisés successivement en faisant varier la pression du fluide gazeux (air) dans l'espace annulaire de 1 (essai A) à 1,5 bars (essai B).

A la sortie dédiée des grains de catalyseur du collecteur d'échantillons, un flacon d'un litre a été installé pour chacune des phases de récupération des matières solides.

Pour chacun de ces deux essais, deux mesures analogues ont été réalisées dans les mêmes conditions (Test 1 et Test 2) avec respectivement un dispositif conforme à la présente invention tel que décrit ci-dessus et un autre dispositif disponible dans l'art et décrit dans FR2566530 et dont les dimensions des deux tubes et du collecteur d'échantillons sont identiques au dispositif conforme à la présente invention.

Pour chacun des essais, le temps de pénétration de la sonde, dans le lit catalytique, d'abord de 0 à 30 cm, puis de 30 à 60 cm, ont été mesurés, la masse de grains de catalyseur récupérée à ensuite été pesée, puis le débit moyen de collecte de grains de catalyseur à été calculé (cf. tableau 1 ci-dessous).

**Tableau 1 : Utilisation du dispositif conforme à l'invention (Test 1) comparativement à un dispositif disponible dans l'art (Test 2) dans un lit catalytique de grains de catalyseur disposé dans un réacteur.**

| **ESSAI A/ Pression air dans l'espace annulaire 1 bar** | | | | | | |
|---|---|---|---|---|---|---|
| | **Pénétration sonde 0 à 30 cm** | | | **Pénétration sonde 30 à 60 cm** | | |
| | **Temps (s)** | **Masse grains de catalyseur collectée (g)** | **Débit collecte échantillons (g/s)** | **Temps (s)** | **Masse grains de catalyseur collectée (g)** | **Débit collecte échantillons (g/s)** |
| Test 1 | 6,5 | 613,1 | 94 | 11,3 | 913,9 | 81 |
| Test 2 | 9,5 | 550,6 | 58 | 22,1 | 697,8 | 32 |

| **ESSAI B/ Pression air dans l'espace annulaire 1,5 bar** | | | | | | |
|---|---|---|---|---|---|---|
| | **Pénétration sonde 0 à 30 cm** | | | **Pénétration sonde 30 à 60 cm** | | |
| | **Temps (s)** | **Masse grains de catalyseur collectée (g)** | **Débit collecte échantillons (g/s)** | **Temps (s)** | **Masse grains de catalyseur collectée (g)** | **Débit collecte échantillons (g/s)** |
| Test 1 | 1,9 | 327,3 | 170 | 9 | 947,1 | 105 |
| Test 2 | 5,5 | 302,8 | 55 | 22,8 | 1054 | 46 |

En effet, les temps de pénétrations de la sonde objet de l'invention sont globalement plus courts d'un facteur variant de 1,5 à 3. Il en est de même pour les débits d'échantillons collectés

Le dispositif conforme à la présente invention et son procédé d'utilisation associé, permet donc de diminuer les temps de manipulation, tout en augmentant les quantités de matières solides prélevées.

## Revendications

1. Dispositif pour l'échantillonnage et/ou la récupération de matières solides empilées à l'état particulaire, comprenant une sonde de prélèvement (1) connectée à un collecteur d'échantillon (2), la sonde (1) comprenant ou étant constituée de :
- au moins deux tubes coaxiaux, un tube interne (3) et un tube externe (4), raccordés étanches à leur partie supérieure (20) et présentant des parties inférieures (13, 14) aptes à pénétrer dans l'empilement des matières solides,
- un espace annulaire (5) entre les deux tubes (3, 4) auquel est connectée une source de fluide sous pression (6),
- une connexion (7) de l'extrémité supérieure du tube interne (3) au collecteur d'échantillons (2) pour y acheminer les particules prélevées grâce notamment à l'action du fluide sous pression (6), le dispositif étant **caractérisé en ce que** la sonde comprend en outre
- une rampe hélicoïdale (19) comprenant au moins une portion de spire (8) fixée et enroulée autour de la partie inférieure externe du tube interne (3) et dont la largeur de la spire (9) est sensiblement égale à la différence des rayons interne du tube externe (4) et externe du tube interne (3).

2. Dispositif selon la revendication 1, **caractérisé en ce que** la longueur entre l'extrémité inférieure (14) du tube externe (4) et l'extrémité inférieure (13) du tube interne (3) est comprise entre 1 mm et 50 mm, et de préférence, entre 1mm et 30 mm.

3. Dispositif selon la revendication précédente, **caractérisé en ce que** l'extrémité inférieure (14) du tube externe (4) est biseautée sur une longueur comprise entre 1 mm et 50 mm, et de préférence comprise entre 5 et 30 mm.

4. Dispositif selon les revendications précédentes, **caractérisé en ce que** le rapport des longueurs entre le rayon interne du tube externe (4) et le rayon externe du tube interne (3) est compris entre 1,1 et 30, et de préférence entre 1,2 et 5.

5. Dispositif selon les revendications précédentes, **caractérisé en ce que** la pression du gaz vecteur est comprise entre 0 bar et 25 bars, et de préférence entre 1 bar et 10 bars.

6. Dispositif selon les revendications précédentes, **caractérisé en ce que** la rampe hélicoïdale (19) peut diviser l'espace annulaire (5) en au moins deux parties, en particulier quatre parties.

7. Dispositif selon la revendication 6, **caractérisé en ce que** la hauteur de la rampe hélicoïdale (19) est comprise entre 50 mm et la hauteur du tube interne (3) recouverte par le tube externe (4), et de préférence entre 150 mm et 500 mm.

8. Dispositif selon les revendications 6 et 7, **caractérisé en ce que** l'épaisseur (9) de la ou des spires (8) est comprise entre 0,1 mm et 50 mm, et de préférence entre 1 mm et 10 mm.

9. Dispositif selon les revendications 6 à 8, **caractérisé en ce que** le pas de la ou des spires (8) est compris entre 10 mm et 300 mm, et de préférence entre 30 et 100 mm.

10. Dispositif selon l'une des revendications 1 à 9, **caractérisé en ce que** la rampe hélicoïdale (19) est montée sur la partie inférieure de du tube interne (3) de manière irréversible.

11. Dispositif selon l'une des revendications 1 à 9, **caractérisé en ce que** la rampe hélicoïdale (19) est montée amovible par rapport à la partie inférieure du tube interne (3), notamment fixée par un pas de vis complémentaire présent sur la partie inférieure du tube interne (3).

12. Dispositif selon les revendications précédentes, **caractérisé en ce que** le tube externe (4) recouvrant la rampe hélicoïdale (19) contient une rainure disposée sensiblement au milieu de 2 spires (8) et dont le pas est sensiblement égal au pas des spires (8).

13. Dispositif selon les revendications précédentes, **caractérisé en ce que** le tube interne (3) comprend au moins une lumière (15) disposée dans son épaisseur.

14. Dispositif selon les revendications précédentes, **caractérisé en ce qu'**il comprend au moins un système destiné à augmenter la vitesse des particules, afin d'améliorer leur acheminement au collecteur (2).

15. Dispositif selon la revendication 14, **caractérisé en ce que** le système est disposé dans la partie supérieure externe du tube interne (3).

16. Dispositif selon les revendications 14 et 15, **caractérisé en ce que** le système est constitué d'une enceinte étanche (16), entourant la partie supérieure du tube interne (3) et alimentée en un fluide sous pression (17).

17. Dispositif selon les revendications 14 à 16, **caractérisé en ce qu'**au moins une lumière (18) est disposée dans l'épaisseur du tube interne (3) à l'intérieur de l'enceinte (16).

18. Procédé pour l'échantillonnage et/ou la récupération de matières solides empilées à l'état particulaire, à l'aide du dispositif selon l'une quelconque des revendications 1 à 17, **caractérisé en ce que** on réalise les opérations suivantes :
- l'extrémité inférieure de la sonde du dispositif est positionnée verticalement ou obliquement sur les matières solides,
- le fluide gazeux est introduit à la pression désirée entre les deux tubes interne et externe,
- les matières solides sont recueillies dans le récipient collecteur dudit dispositif au fur et à mesure que ladite sonde pénètre dans l'empilement,
- la fin du prélèvement, c'est à dire l'arrêt de la pénétration de la sonde est obtenue par coupure de l'alimentation en fluide gazeux.

19. Utilisation du dispositif selon l'une quelconque des revendications 1 à 17 ou du procédé selon la revendication 18 pour le prélèvement d'échantillons dans un empilement de matières solides disposées à l'état particulaire en une ou plusieurs couches dans une atmosphère gazeuse ou liquide.

## Patentansprüche

1. Vorrichtung zur Probenahme und/oder Rückgewinnung von im Partikelzustand gestapelten Feststoffen, umfassend eine Entnahmesonde (1), die mit einem Probensammler (2) verbunden ist, wobei die Sonde (1) umfasst oder gebildet ist von:
- mindestens zwei koaxialen Rohren, einem inneren Rohr (3) und einem äußeren Rohr (4), die an ihrem oberen Teil (20) dicht verbunden sind und untere Teile (13, 14) aufweisen, die imstande sind, in den Stapel der Feststoffe einzudringen,
- einem ringförmigen Raum (5) zwischen den zwei Rohren (3, 4), mit dem eine Quelle von Fluid unter Druck (6) verbunden ist,
- einer Verbindung (7) des oberen Endes des inneren Rohrs (3) mit dem Probensammler (2), um dort die dank vor allem der Wirkung des Fluids unter Druck (6) entnommenen Partikel zu befördern,
wobei die Vorrichtung **dadurch gekennzeichnet ist, dass** die Sonde ferner umfasst:
- eine schraubenförmige Rampe (19), die mindestens einen Windungsabschnitt (8) umfasst, der um den äußeren unteren Teil des inneren Rohrs (3) befestigt und gewickelt ist und dessen Breite der Windung (9) etwa gleich der Differenz des inneren Radius des äußeren Rohrs (4) und des äußeren Radius des inneren Rohrs (3) entspricht.

2. Vorrichtung nach Anspruch 1, **dadurch gekennzeichnet, dass** die Länge zwischen dem unteren Ende (14) des äußeren Rohrs (4) und dem unteren Ende (13) des inneren Rohrs (3) zwischen 1 mm und 50 mm und vorzugsweise zwischen 1 mm und 30 mm inklusive beträgt.

3. Vorrichtung nach vorangehendem Anspruch, **dadurch gekennzeichnet, dass** das untere Ende (14) des äußeren Rohrs (4) über eine Länge zwischen 1 mm und 50 mm inklusive und vorzugsweise zwischen 5 und 30 mm inklusive abgeschrägt ist.

4. Vorrichtung nach den vorangehenden Ansprüchen, **dadurch gekennzeichnet, dass** das Verhältnis der Längen zwischen dem inneren Radius des äußeren Rohrs (4) und dem äußeren Radius des inneren Rohrs (3) zwischen 1,1 und 30 und vorzugsweise zwischen 1,2 und 5 beträgt.

5. Vorrichtung nach den vorangehenden Ansprüchen, **dadurch gekennzeichnet, dass** der Druck des Vektorgases zwischen 0 bar und 25 bar und vorzugsweise zwischen 1 bar und 10 bar inklusive ist.

6. Vorrichtung nach den vorangehenden Ansprüchen, **dadurch gekennzeichnet, dass** die schraubenförmige Rampe (19) den ringförmigen Raum (5) in mindestens zwei Teile, insbesondere vier Teile, unterteilen kann.

7. Vorrichtung nach Anspruch 6, **dadurch gekennzeichnet, dass** die Höhe der schraubenförmigen Rampe (19) zwischen 50 mm und der Höhe des von dem äußeren Rohr (4) bedeckten inneren Rohrs (3) und vorzugsweise zwischen 150 mm und 500 mm inklusive beträgt.

8. Vorrichtung nach den Ansprüchen 6 und 7, **dadurch gekennzeichnet, dass** die Dicke (9) der Windung(en) (8) zwischen 0,1 mm und 50 mm und vorzugsweise zwischen 1 mm und 10 mm inklusive beträgt.

9. Vorrichtung nach den Ansprüchen 6 bis 8, **dadurch gekennzeichnet, dass** die Steigung der Windung(en) (8) zwischen 10 mm und 300 mm und vorzugsweise zwischen 30 und 100 mm inklusive beträgt.

10. Vorrichtung nach einem der Ansprüche 1 bis 9, **dadurch gekennzeichnet, dass** die schraubenförmige Rampe (19) am unteren Teil des inneren Rohrs (3) irreversibel angebracht ist.

11. Vorrichtung nach einem der Ansprüche 1 bis 9, **dadurch gekennzeichnet, dass** die schraubenförmige Rampe (19) in Bezug zum unteren Teil des inneren Rohrs (3) lösbar angebracht ist, vor allem mittels eines komplementären Schraubengangs, vorliegend im unteren Teil des inneren Rohrs (3), befestigt ist.

12. Vorrichtung nach den vorangehenden Ansprüchen, **dadurch gekennzeichnet, dass** das die schraubenförmige Rampe (19) bedeckende äußere Rohr (4) eine Rille enthält, die etwa in der Mitte von zwei Windungen (8) angeordnet ist und deren Steigung etwa der Steigung der Windungen (8) entspricht.

13. Vorrichtung nach den vorangehenden Ansprüchen, **dadurch gekennzeichnet, dass** das innere Rohr (3) mindestens ein Lumen (15) umfasst, die in seiner Dicke angeordnet ist.

14. Vorrichtung nach den vorangehenden Ansprüchen, **dadurch gekennzeichnet, dass** sie mindestens ein System umfasst, das bestimmt ist, die Geschwindigkeit der Partikel zu erhöhen, um ihre Beförderung zum Sammler (2) zu verbessern.

15. Vorrichtung nach Anspruch 14, **dadurch gekennzeichnet, dass** das System im äußeren oberen Teil des inneren Rohrs (3) angeordnet ist.

16. Vorrichtung nach den Ansprüchen 14 und 15, **dadurch gekennzeichnet, dass** das System von einem dichten Behälter (16) gebildet ist, der den oberen Teil des inneren Rohrs (3) umgibt und mit einem Fluid unter Druck (17) versorgt wird.

17. Vorrichtung nach den Ansprüchen 14 bis 16, **dadurch gekennzeichnet, dass** mindestens ein Lumen (18) in der Dicke des inneren Rohrs (3) im Innern des Behälters (16) angeordnet ist.

18. Verfahren für die Probenahme und/oder die Rückgewinnung von im Partikelzustand gestapelten Feststoffe mit Hilfe der Vorrichtung nach einem der Ansprüche 1 bis 17, **dadurch gekennzeichnet, dass** die folgenden Maßnahmen durchgeführt werden:
- Positionieren des unteren Endes der Sonde der Vorrichtung vertikal oder schräg auf den Feststoffen,
- Einleiten des gasförmigen Fluids mit dem gewünschten Druck zwischen die zwei, das innere und das äußere, Rohre,
- Sammeln der Feststoffe im Sammelbehälter der Vorrichtung nach und nach, wie die Sonde in den Stapel eindringt,
- Unterbrechen der Versorgung mit gasförmigem Fluid, um die Entnahme zu beenden, das heißt, das Eindringen der Sonde zu stoppen.

19. Verwendung der Vorrichtung nach einem der Ansprüche 1 bis 17 oder des Verfahrens nach Anspruch 18 für die Probenahme in einem Stapel von Feststoffen, angeordnet im Partikelzustand in einer oder in mehreren Schichten in einer gasförmigen oder flüssigen Atmosphäre.

## Claims

1. Device for sampling and/or recovering solid material stacked in particulate state, comprising a sampling probe (1) connected to a sample collector (2), the probe (1) comprising or consisting of:
- at least two coaxial tubes, an inner tube (3) and an outer tube (4), sealingly connected at their upper part (20) and having lower parts (13, 14) able to enter into the stack of solid material,
- an annular space (5) between the two tubes (3, 4) to which a pressurized fluid source (6) is connected,
- a connection (7) of the upper end of the inner tube (3) to the sample collector (2) so as to convey therein the particles sampled by means of the action in particular of the pressurized fluid (6),
the device being **characterized in that** the probe further comprises:
- a helical ramp (19) comprising at least one coil portion (8) attached to and wound around the lower outer part of the inner tube (3), and whose width of the coil (9) is substantially equal to the difference between the inner radius of the outer tube (4) and outer radius of the inner tube (3).

2. The device according to claim 1, **characterized in that** the length between the lower end (14) of the outer tube (4) and the lower end (13) of the inner tube (3) is between 1 mm and 50 mm, and preferably between 1 mm and 30 mm.

3. The device according to the preceding claim, **characterized in that** the lower end (14) of the outer tube (4) is bevelled over a length of between 1 mm and 50 mm, and preferably of between 1 mm and 30 mm.

4. The device according to the preceding claims, **characterized in that** the ratio of the lengths between the inner radius of the outer tube (4) and the outer radius of the inner tube (3) is between 1.1 and 30, and preferably between 1.2 and 5.

5. The device according to the preceding claims, **characterized in that** the pressure of the vector gas is between 0 bar and 25 bars, and preferably between 1 bar and 10 bars.

6. The device according to the preceding claims, **characterized in that** helical ramp (19) may divide the annular space (5) into at least two parts, in particular into four parts.

7. The device according to claim 6, **characterized in that** the height of the helical ramp (19) is between 50 mm and the height of the inner tube (3) covered by the outer tube (4), and preferably between 150 mm and 500 mm.

8. The device according to claims 6 and 7, **characterized in that** the thickness (9) of the coil(s) (8) is between 0.1 mm and 50 mm, and preferably between 1 mm and 10 mm.

9. The device according to claims 6 to 8, **characterized in that** the pitch of the coil(s) (8) is between 10 mm and 300 mm, and preferably between 30 and 100 mm.

10. The device according to one of claims 1 to 9, **characterized in that** the helical ramp (19) is irreversibly mounted on the lower part of the inner tube (3).

11. The device according to one of claims 1 to 9, **characterized in that** the helical ramp (19) is removably mounted relative to the lower part of the inner tube (3), in particularly attached by a mating screw thread on the lower part of the inner tube (3).

12. The device according to the preceding claims, **characterized in that** the outer tube (4) covering the helical ramp (19) contains a groove arranged substantially in the centre of 2 coils (8) and having a pitch substantially equal to the pitch of the coils (8).

13. The device according to the preceding claims, **characterized in that** the inner tube (3) comprises at least one lumen (15) arranged in the thickness thereof.

14. The device according to the preceding claims, **characterized in that** it comprises at least one system for increasing the velocity of the particles, to improve the conveying thereof towards the collector (2).

15. The device according to claim 14, **characterized in that** the system is arranged in the upper outer part of the inner tube (3).

16. The device according to claims 14 and 15, **characterized in that** the system is formed of a sealed enclosure (16) surrounding the upper part of the inner tube (3) and supplied with a pressurized fluid (17).

17. The device according to claims 14 to 16, **characterized in that** at least one lumen (18) is arranged in the thickness of the inner tube (3) inside the enclosure (16).

18. Method for sampling and/or recovering solid material stacked in particulate state, using the device according to any of claims 1 to 17, **characterized in that** the following operations are performed:
- the lower end of the probe of the device is positioned vertically or obliquely on the solid material,
- the gaseous fluid is inserted at the desired pressure between the two inner and outer tubes,
- the solid material is collected in the collecting container of said device as and when said probe penetrates the stack,
- the end of sampling i.e. the stopping of the penetration of the probe is obtained by cutting off the supply of gaseous fluid.

19. Use of the device according to any of claims 1 to 17 or of the method according to claim 18 to take samples from a stack of solid material stacked in particulate state in one or more layers in a gaseous or liquid atmosphere.
